# EUROPEAN PATENT APPLICATION

(11) **EP 2 845 863 A1**
(43) Date of publication of application: **11.03.2015**
(21) Application number: 13382350.0
(22) Date of filing: 10.09.2013
(51) Int. Cl.: C07K 14/195

(54) **Mathanococcus jannaschii protease and its use**

(71) Applicant: Consejo Superior de Investigaciones Cientificas (CSIC), 28006 Madrid (ES)
(72) Inventor: Cerdà Costa, Núria, 28006 Madrid (ES); López Pelegrin, Mar, 08028 Barcelona (ES); Cintas Pedrola, Anna, 08028 Barcelona (ES); López Arolas, Joan, 08028 Barcelona (ES); Gomis-Rüth, Francesc Xavier, 08028 Barcelona (ES)
(74) Representative: Oficina Ponti, SLP

(57) **Abstract**

The present invention relates to a proteolytic enzyme having the amino acid sequence SEQ ID No: 1 and a process for obtaining thereof. The present invention further relates to an isolated nucleic acid molecule encoding said proteolytic enzyme. The present invention further relates to several uses of said proteolytic enzyme.

SEQUENCE LISTING

<110> Consejo Superior de Investigaciones Científicas (CSIC)

<120> Novel proteolytic enzyme for specific cleavage and use thereof

<130> P26977EP00

<160> 1

<170> PatentIn version 3.5

<210> 1

<211> 110

<212> PRT

<213> Methanococcus jannaschii

<400> 1

Met Lys Asp Arg Lys Ile Leu Asn Glu Ile Leu Ser Asn Thr Ile Asn
1               5                   10                  15

Glu Leu Asn Leu Asn Asp Lys Lys Ala Asn Ile Lys Ile Lys Ile Lys
            20                  25                  30

Pro Leu Lys Arg Lys Ile Ala Ser Ile Ser Leu Thr Asn Lys Thr Ile
        35                  40                  45

Tyr Ile Asn Lys Asn Ile Leu Pro Tyr Leu Ser Asp Glu Glu Ile Arg
    50                  55                  60

Phe Ile Leu Ala His Glu Leu Leu His Leu Lys Tyr Gly Lys Tyr His
65                  70                  75                  80

Ile Asn Glu Phe Glu Glu Glu Leu Leu Phe Leu Phe Pro Asn Lys Glu

                85                  90                  95

Ala Ile Leu Ile Asn Leu Ile Asn Lys Leu His Gln Lys Lys

            100                 105                 110

SEQUENCE LISTING

<110>  Consejos Superior de Investigaciones Científicas (CSIC)

<120>  Novel proteolytic enzyme for specific cleavage and use thereof

<130>  P26977EP00

<160>  1

<170>  PatentIn version 3.5

<210>  1
<211>  110
<212>  PRT
<213>  Methanococcus jannaschii

<400>  1

Met Lys Asp Arg Lys Ile Leu Asn Glu Ile Leu Ser Asn Thr Ile Asn
1               5                   10                  15

Glu Leu Asn Leu Asn Asp Lys Lys Ala Asn Ile Lys Ile Lys Ile Lys
            20                  25                  30

Pro Leu Lys Arg Lys Ile Ala Ser Ile Ser Leu Thr Asn Lys Thr Ile
        35                  40                  45

Tyr Ile Asn Lys Asn Ile Leu Pro Tyr Leu Ser Asp Glu Glu Ile Arg
    50                  55                  60

Phe Ile Leu Ala His Glu Leu Leu His Leu Lys Tyr Gly Lys Tyr His
65                  70                  75                  80

Ile Asn Glu Phe Glu Glu Glu Leu Leu Phe Leu Phe Pro Asn Lys Glu
                85                  90                  95

Ala Ile Leu Ile Asn Leu Ile Asn Lys Leu His Gln Lys Lys
            100                 105                 110

## Description

### Field of the invention

The present invention relates to the field of enzymes. In particular the present invention relates to a new proteolytic enzyme designated herein as "selecase".

### Background of the invention

Proteolytic enzymes (also known as peptidases, proteases or proteinases) have many applications in biotechnology and biomedicine owing to their capacity to cleave peptide bonds of proteins and peptides. However, most peptidases are not specific and cleave various substrates, usually at multiple positions. A common substrate used to test the preferred cleavage sites of a given peptidase in standard biochemical assays is bovine milk casein. To date, peptidases from all catalytic types (i.e. serine, aspartic, cysteine, and metallopeptidases) have been shown to cleave casein, mostly at multiple positions [1].

Casein (from Latin caseus, "cheese") is a family of related phosphoproteins (chains αₛ₁, αₛ₂, β, and κ) that are commonly found in mammalian milk, making up 80% of the proteins in bovine milk. Casein can be found in different food products, either as a component of milk (milk-derived products like cheese and yogurt) or as an additive (baked products, meat, broths). It has been largely proven that certain proteolytic cleavages on milk-derived products are able to change their properties [2]. For instance, the aspartic peptidase chymosin (also known as rennin) is commonly used in the lactic industry to cleave peptide bond F126-M127 of the κ-chain of bovine milk casein (see UniProt [UP] sequence database access code P02668) triggering milk coagulation to form curds, which afterwards can be compressed into cheese. Peptidases also affect the flavor and organoleptic characteristics of cheese (e.g. they can provide a meaty, appetizing flavor and reduce unwanted bitterness) [3]. Furthermore, chains α and β of casein can act as molecular chaperones, interacting with 'molten globule' states or folding intermediates of milk proteins and stabilizing them against aggregation and precipitation under conditions of stress, e.g. during pasteurization.

Casein has also been shown to induce the immune response mediated by IgE when administered to selected individuals. In fact, bovine milk αₛ₁-casein (UP P02662) is also referred to as *allergen Bos d 8.* One of the epitopes responsible for the allergenicity of this casein chain spans residues N154-F168, which is found with ∼93% sequence homology in milks from other mammals like goat or sheep. Proteolysis has been proven to be a helpful tool in the decrease of the immuno-reactivity of this protein [4].

Besides its use in lactic and food industries, over the years casein has been used as a biomaterial in many non-food related industrial applications, such as leather, glues, and biofilms, due to its primary and secondary structure characteristics and the advantages that arise from those [5,6]. Caseins are mostly random coil polypeptides with a high degree of molecular flexibility. The typical intermolecular interactions that they can establish confer on them good film-forming and coating abilities. When applied as a thin layer on the surface of a material, casein increases its affinity for pigments, its ink-binding properties and its adhesion to various substrates, such as wood, glass or paper, allowing its use in paper industry and as glue for adhesives in the bottling industry. Moreover, casein's amphipathic nature provides it with good emulsifying and stabilizing properties and makes it suitable for being used as an additive in paint, concrete formulations, and cosmetics. Casein can also act as a barrier for apolar gases (O₂ and CO₂) and aromas. Accordingly, casein-based films and biopolymers, which are biodegradable, are an emerging alternative in the packaging field.

In addition, casein plays a role in the formation of biofilms, which are surface-attached agglomerations of bacteria at infection sites such as in the gums. Biofilm formation is often observed in bacteria that colonize or infect humans or animals, and the capability of certain bacteria strains to produce biofilms relates to its virulence such as in periodontitis. As a result, the most frequent biofilm-related infections are nosocomial infections on implanted or close-contact medical devices (catheters, artificial valves, etc), where the physicochemical properties of the material being colonized are crucial. Biofilms can be disrupted by mechanical or chemical forces, and peptidases play an important role in the detachment of bacteria to a colonized surface. It has been recently reported that biofilm formation in bovine mastitis-related bacterial strains can be induced by the presence of casein proteins; in particular αₛ₁-casein has the highest potential to facilitate bacterial biofilm formation [7].

In recent years, much effort has focused on casein-derived bioactive peptides that have been proven to exert numerous biological effects in the body and may ultimately have an influence on health. Consequently, these specific peptides have interesting applications as supplements in food (e.g. cheese and yogurt) and pharmaceutical preparations. Thus, a relatively high number of bioactive peptides derived from the hydrolysis of casein from mammalian milk have been described to display a variety of properties like anti-carcinogenic or sedative [8]. Other bioactive peptides possess immuno-modulatory effects or play a role in the transport and absorption of certain minerals. Casein-derived peptides are not active within the "parent" protein but are released and activated during enzymatic hydrolysis, microbial fermentation, or gastrointestinal digestion.

Another biotechnological application of peptidases is related to cleavage of recombinant fusion tags. Advances in DNA technology have facilitated the recombinant overexpression of a wide range of genes in different host organisms, such as bacteria and yeast. This has resulted in massive use of recombinant proteins that consist of peptides and proteins of interest coupled to a fusion protein or tag that can act as folding assistant-e.g. by increasing the solubility of the target protein-; prevent its degradation in host cells; simplify purification by enabling affinity chromatography (e.g. His-tag, Flag-tag, GST, MBP, etc.); and allow tracing during purification-the fusion protein acts as a reporter of the localization of the target protein in different cell compartments [9]. However, it is advisable to remove the fusion construct once it has performed its function in order to obtain the target protein without extra residues attached or with just one or two, i.e. close to its native state. Chemical methods can be used for such tag removal but they frequently require elevated temperatures and toxic compounds, which denature the target protein and may lead to undesired non-specific degradation. The use of a peptidase that recognizes a highly specific sequence is therefore of great interest, since it can assure the removal of the fusion tag while maintaining the integrity of the target protein (the probabilities of finding the specific stretch of peptide sequence inside a given protein are minimal). Many peptidases such as factor Xa, enterokinase, and thrombin have been used for this purpose, although they are not highly specific and cleavage can occur inside the target protein at additional sites distinct from the recognition sequence [10]. Only a few peptidases such as tobacco etch virus proteinase (TEV proteinase) or human rhinovirus 3C proteinase (PreScission proteinase) are widely available nowadays to achieve a high level of cleavage specificity [11]. However, even these reagents have limitations, such as low efficiency in the presence of chaotropic agents or detergent; the need for reducing agents in the reaction mixture, which negatively affects the integrity of disulfide bonds of the target protein; autolysis; and limited temperature range of application. Therefore, there is still a need in the art for proteolytic enzymes capable of specifically cleaving off fusion constructs.
The present invention provides a new proteolytic enzyme termed selecase, which is able to specifically cleave the peptide bond Y159-F160 of the αₛ₁-chain of casein (E-L-A-Y-↓-F-Y-P-E) as well as fusion tags of recombinant proteins that contain the sequence E-X-X-Y-↓-F (X for any residue). In particular, it cleaves within the TEV proteinase cleavage sequence (E-N-L-Y-↓-F-Q-S/G) but at a different site (TEV proteinase cleaves before S/G). Selecase has many different applications as disclosed below in detail.

### Brief description of drawings

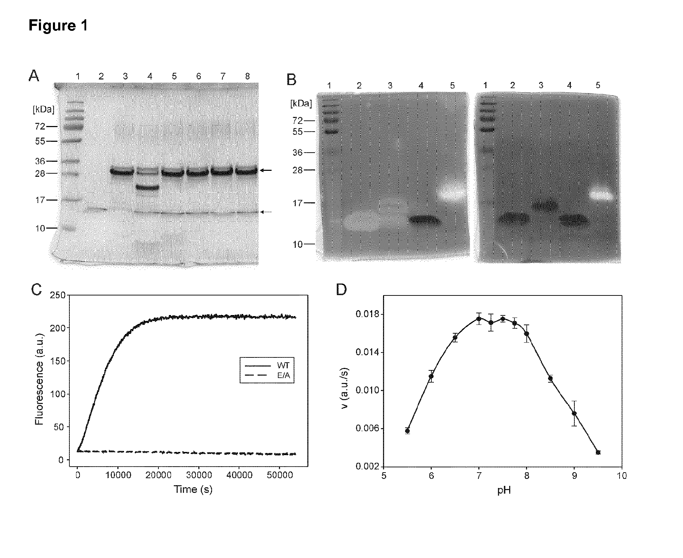

**Figure 1****. Metalloproteinase activity of selecase. (A)** Coomassie-stained SDS-PAGE of α-casein incubated overnight at 37°C with selecase (at 25 µg/ml: 1:20 peptidase:substrate molar ratio). Lane 1, molecular mass standard (PageRuler Plus Prestained Protein Ladder; Fermentas) ranging from 10 to 250 KDa. Lane 2, selecase. Lane 3, intact α-casein consisting of αₛ₂-casein (top light band; 25 KDa) and αₛ₁-casein (bottom strong band; 22-23.7 KDa). Lane 4, α-casein incubated with wild-type selecase. Only αₛ₁-casein is cleaved. Lanes 5, 6, 7, and 8, α-casein incubated, respectively, with the catalytically inactive selecase mutant E⁷⁰A and the wild-type peptidase in the presence of 1 mM EDTA, 1 mM ZnCl₂, and 1 mM 1,10-phenanthroline. The positions of intact αₛ₁/αₛ₂-casein and selecase are indicated by arrows. **(B)** Coomassie-stained zymogram of α-casein incubated overnight at 37°C loaded with selecase samples in the absence (left panel) and in the presence (right panel) of 5 mM 1,10-phenanthroline. Caseinolytic activity is detected as light lysis zones. Lane 1, molecular mass standard. Lane 2, wild-type selecase (8 µg). Lane 3, wild-type selecase (8 µg) with an N-terminal hexahistidine fusion tag followed by a TEV proteinase recognition site, thus resulting in a larger, ∼17-KDa protein. Part of this protein undergoes autolytic cleavage at position E-N-L-Y-↓-F-Q-G of the TEV proteinase cleavage site, thus resulting in two bands (left gel). Lane 4, E⁷⁰A selecase mutant (8 µg). Lane 5, bovine trypsin (0.08 µg) as positive control. **(C)** Proteolysis of the fluorogenic peptide Abz-E-L-A-Y-↓-F-Y-P-E-K(dnp) at 10 µM over time by incubation at 37°C in 50 mM HEPES, 150 mM NaCl, pH 7.5, with either wild-type (WT) or the E⁷⁰A mutant (E/A) of selecase at 2 µM. **(D)** Activity dependence of wild-type selecase on pH as determined with the fluorogenic peptide Abz-E-L-A-Y-↓-F-Y-P-E-K(dnp) as substrate (see "Example section" for details).

### Summary of the invention

In a first aspect, the present invention relates to a proteolytic enzyme as defined below in the present invention.

In a second aspect, the present invention relates to an isolated nucleic acid molecule encoding a proteolytic enzyme according to the first aspect of the invention.

In a third aspect, the present invention relates to a recombinant expression vector comprising the isolated nucleic acid molecule according to the second aspect of the invention.

In a fourth aspect, the present invention relates to a host cell comprising the recombinant expression vector according to the third aspect of the invention.

In a fifth aspect, the present invention relates to a process for producing a proteolytic enzyme according to the first aspect of the invention.

In a sixth aspect, the present invention relates to an enzyme preparation comprising a proteolytic enzyme according to the first aspect of the invention and a process for obtaining thereof.

In a seventh aspect, the present invention relates to several uses of a proteolytic enzyme according to the first aspect of the invention.

### Detailed description of the invention

The present invention relates to a new proteolytic enzyme, particularly a metalloproteinase, designated herein as selecase, characterized in that said protease has the amino acid sequence (SEQ ID No: 1) shown below under SEQUENCE LISTING; or an amino acid sequence having at least 70%, more preferably 80%, even more preferably, 90%, further more preferably 95% identity to the amino acid sequence of the proteolytic enzyme as defined as SEQ ID No: 1 and optionally has additional N-terminal and/or C-terminal residues coming from fusion constructs with other proteins and/or tags.

As most metalloproteinases, selecase possesses a short consensus zinc-binding sequence (here H⁶⁹EXXH⁷³), which is indispensable for catalysis [14]. Consistently, mutant E⁷⁰A mutant (see SEQ ID No: 1), which affects the glutamate that plays an essential role as a general base/acid in the catalytic process of metallopeptidases in general [14], is inactive against bovine milk αₛ₁-casein (see Fig. 1). However, mutations at other positions, which do not affect the general scaffold of the protein, might not hamper proteolytic activity of selecase and eventually could even improve its biophysical properties (e.g. solubility, thermostability, catalytic efficiency, etc).

In a preferred embodiment, said protease enzyme is obtainable from *Methanococcus jannaschii.*

The present invention also relates to an isolated nucleic acid molecule encoding a new proteolytic enzyme designated herein as selecase selected from the group consisting of:
(a) a nucleic acid molecule encoding a polypeptide comprising the amino acid sequence as depicted in SEQ ID No: 1;
(b) a nucleic acid molecule encoding a polypeptide having at least 70%, more preferably 80%, even more preferably, 90%, further more preferably 95% identity to the amino acid sequence of the proteolytic enzyme as defined in SEQ ID No: 1;
(c) a nucleic acid molecule comprising the coding sequence of the nucleotide sequence as depicted in SEQ ID No: 1 plus N-terminal and C-terminal fusion proteins or tags;
(d) a nucleic acid molecule the coding sequence of which differs from the coding sequence of a nucleic acid molecule of (c) due to the degeneracy of the genetic code; and
(e) a nucleic acid molecule hybridizing under stringent conditions to a nucleic acid molecule encoding a polypeptide having an amino acid sequence, which shows at least 70%, more preferably 80%, even more preferably, 90%, further more preferably 95% identity to the amino acid sequence as depicted in SEQ ID No: 1.

The present invention further relates to a recombinant expression vector comprising the isolated nucleic acid molecules listed under a-e above, operably linked to regulatory sequences capable of directing expression of said selecase encoding gene in a suitable host. Suitable hosts include all strains of *Escherichia coli, Bacillus* spp., *Pichia pastoris, Saccharomyces cerevisiae,* insect and mammalian cells, and *Methanococcus* spp.

The present invention relates to a host cell comprising the recombinant expression vector as described above. Preferably, the host cells include all strains of *Escherichia coli, Bacillus* spp., *Pichia pastoris, Saccharomyces cerevisiae,* insect and mammalian cells, and *Methanococcus* spp.

The present invention relates to a process of producing a proteolytic enzyme according to the present invention, said process comprising the steps of culturing the host cell of the invention and isolation/purification of the proteolytic enzyme. Also within the invention is a proteolytic enzyme encoded by the nucleic acid sequence of the invention (see a-e, above) and which is obtainable by the process described above.

The invention relates to a process for obtaining an enzyme preparation comprising the proteolytic enzyme of the present invention, said process comprising the steps of culturing a host cell of the invention and either recovering the proteolytic enzyme of the invention from the cells or separating the cells from the culture medium and obtaining the supernatant. Within the invention is also an enzyme preparation obtainable by the process described above.

The invention further relates to an enzyme preparation, which comprises the proteolytic enzyme of the invention.

The enzyme preparation of the invention may further comprise other enzymes selected from the group of proteases, amylases, cellulases, lipases, xylanases, mannanases, cutinases, pectinases or oxidases, with or without a mediator, as well as suitable additives selected from the group of stabilizers, buffers, surfactants, bleaching agents, mediators, anti-corrosion agents, builders, antiredeposition agents, optical brighteners, dyes, pigments, caustics, abrasives and preservatives, etc.

By the term **"identity"**, herein the identity between two amino acid sequences is meant when aligned in a frame so that the maximal number of residues coincide. For example, the identity of a full-length or a mature sequence of two proteins or peptides with their associated amino-acid sequences may be computed. The amino-acid sequences of two molecules to be compared may differ in none, one or more positions, which however do not necessarily alter the biological function or structure of the molecules. Such variation may occur naturally because of different host organisms or mutations in the amino-acid sequence or they may be achieved by specific mutagenesis. The variation may result from deletion, substitution, insertion, addition or combination of one or more positions in the amino acid sequence.

The percentage of identity between two amino acid sequences in this invention designates the percentage of residues of identical amino acids between the two sequences that are compared, which is produced after achieving the best alignment, and the percentage being purely statistical, and the differences between the two sequences may be randomly distributed and throughout the sequence. The best alignment relates to the alignment for which the percentage of identity is the greater.

The comparison between two amino acid sequences can be made, for example, using the blastP computer program which is available on the website (http://www.ncbi.nlm.nih.gov/BLAST/) of the National Center for Biotechnology Information.

The percentage of identity between two amino acid sequences is calculated by firstly comparing the two sequences positioned according to the best alignment, and determining the number of identical positions for which the residue of amino acids is identical between the sequences.

The percentage of identity between the two sequences compared is calculated by dividing this number of identical positions by the total number of compared positions, and multiplying the result by 100.

A polypeptide which has a certain percentage of identity with another one, is typically designated as a homologous polypeptide.

**"Stringency"** of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures.

Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so.

**"Stringent conditions" or "high stringency conditions"**, as defined herein, can be identified by those that: (1) employ low ionic strength and high temperature for washing, for example 15 mM NaCl/1.5 mM sodium citrate/0.1% SDS at 50°C; (2) employ during hybridization a denaturing agent, such as formamide, for example, 50% (v/v) formamide with 0.1% BSA/0.1% Ficoll/0.1% polyvinylpyrrolidone/50 mM sodium phosphate buffer at pH 6.5 with 0.75 M NaCl, 75 mM sodium citrate at 42°C; or (3) overnight hybridization in a solution that employs 50% formamide, 5x SSC (0.75 M NaCl, 75 mM sodium citrate), 50 mM sodium phosphate (pH 6.8), 0.1% sodium pyrophosphate, 5x Denhardt's solution, sonicated salmon sperm DNA (50 mg/ml), 0.1% SDS, and 10% dextran sulfate at 42°C, with a 10 min wash at 42°C in 0.2x SSC (NaCl/sodium citrate) followed by a 10 min high-stringency wash consisting of 0.1x SSC containing EDTA at 55°C.

The term **"control sequences"** refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is **"operably linked"** when it is placed into a functional relationship with another nucleic acid sequence. For example, DNA for a pre-sequence or secretory leader is operably linked to DNA for a polypeptide if it is expressed as a pre-protein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. In view of the background art, the proteolytic enzyme provided herein (selecase) has been shown to be able to specifically cleave the peptide bond Y159-F160 within bovine αₛ₁-casein molecule and related sequences regardless of their origin (e.g. synthetic polypeptide chains, flurorogenic peptides, regular peptides), conferring thereby novel organoleptic properties to lactic products and derived food additives (e.g. flavor and/or texture variations), or improving the digestibility and solubility of protein hydrolysates.

At the same time other important effects are provided by the new proteolytic enzyme disclosed herein:
- In food industry by modifying the properties of milk-derived products and reduction/avoidance of milk/milk-derived products allergenicity. The cleavage of the peptide bond Y159-F160 would trigger the destruction of one of the epitopes responsible of the allergenicity of αₛ₁-casein and the anaphylactic response could thus be diminished or even completely avoided.
- In biomaterials by modifying casein-derived biomaterials properties and disruption of bacterial biofilms. The proteolytic activity of selecase would alter the physical properties of products such as casein-based films and biopolymers, making them more suitable and/or attractive for their specific applications. Its selective cleavage would also help to disrupt the biofilm formation and be of potential aid in combating biofilm-mediated bacterial infections via direct application or by engineered selecase-coated instruments (alone or in combination with other peptidases) as a profilactic measure.
- In generation of novel casein-derived bioactive peptides by cleaving casein in a new position.

Importantly, selecase is the smallest reported active proteolytic enzyme (110 residues in comparison to 200-400 residues of most single-domain peptidases), it is thermostable (it is from the thermophilic archaeon *Methanococcus jannaschii),* and it shows a unique mechanism of self-inhibition at increasing protein concentrations. In addition, selecase is proteolytically active in a wide range of pH (5.5-9.0), and owing to its thermostable nature, it is active at high temperatures (up to 65°C). Therefore, selecase is a novel alternative for the field of peptidases with many advantages: it is a small peptidase that is easily expressed and purified, and stored; it is capable of working at high temperatures; and it does not have any special requirements for function, such as the need for reducing agents.

In addition, the specific ability of selecase to cleave at position Y↓F of the peptide sequence E-L-A-Y-↓-F-Y-P-E (i.e. the peptide bond Y159-F160 of bovine αₛ₁-casein) is important for its application in recombinant protein production. It enables removal of a fusion protein or tag in which the peptide sequence between the fusion protein and the target protein contains this peptide sequence or related ones.

In a further aspect, the present invention also relates to the use of the proteolytic enzyme of the present invention or the enzyme preparation of the present invention in biomaterials for modifying casein-derived biomaterials properties and disruption of bacterial biofilms.

The present invention also relates to the use of the proteolytic enzyme of the present invention or the enzyme preparation of the present invention in the food industry for modifying the properties of milk-derived products and reduction/avoidance of milk/milk-derived products allergenicity.

The present invention further relates to the use of the proteolytic enzyme of the present invention or the enzyme preparation of the present invention in the generation of novel peptides.

The present invention further relates to the use of the proteolytic enzyme of the present invention or the enzyme preparation of the present invention in recombinant protein expression for removal of a fusion protein, in which the peptide sequence between the fusion protein and the target protein contains this peptide sequence or related ones.

The following Examples are offered for illustrative purposes only and are not intended to limit the scope of the present invention in any way.

### EXAMPLE 1: Process of obtaining selecase

1) The gene coding for selecase (SEQ ID No: 1 depicts its amino-acid sequence) was amplified from *Methanococcus jannaschii* genomic DNA and cloned into vector pPROEX-HTa (Invitrogen; resistant towards ampicillin), which attaches an N-terminal hexahistidine(His₆)-tag followed by a tobacco etch virus (TEV) proteinase recognition site, using BamHI and *Hind*III restriction sites. The gene was also cloned into vector pCRI7 (modified from pET28a vector, Novagen, confers resistance towards kanamycin), which does not attach extra residues, using *Nde*I and *Xho*I restriction sites. A single-residue point mutant (E⁷⁰A mutant) was generated using the QuikChange Site-Directed Mutagenesis kit (Stratagene) according to the manufacturer's instructions. The constructs were verified by DNA sequencing.
2) Selecase and its mutant were produced by heterologous overexpression in *Escherichia coli* BL21 (DE3) cells, which were grown at 37°C in Luria Bertani medium supplemented with either 100 µg/ml ampicillin (pPROEX-HTa vector) or 30 µg/ml kanamycin (pCRI7 vector). Cultures were induced at an OD₆₀₀ of 0.8 with 1 mM isopropyl-β-D-thiogalactopyranoside and kept growing for 5 h at 37°C.
3) Selecase and its mutant were purified as follows. After centrifugation at 7,000xg for 30 min at 4°C, the pellet was washed twice with buffer A (50 mM Tris-HCl, 500 mM NaCl, pH 8.0) and resuspended in the same buffer supplemented with EDTA-free protease inhibitor cocktail tablets and DNase I (both Roche Diagnostics). Cells were lysed using a cell disrupter (Constant Systems) at a pressure of 1.35 Kbar, and the cell debris was removed by centrifugation at 50,000xg for 1 h at 4°C. Next purification steps depended on the presence or absence of His₆-tag: (i) The supernatant of the His₆-tagged protein (encoded by vector pPROEX-HTa) was filtered (0.22 µm pore size; Millipore), loaded onto a HiTrap Chelating HP column (GE Healthcare) charged with nickel chloride, and the fusion protein was eluted using a gradient of buffer A plus 10-500 mM imidazole. Subsequently, the sample was dialyzed overnight at 20°C against buffer B (50 mM Tris-HCl, 250 mM NaCl, 1 mM dithiothreitol, pH 8.0) in the presence of His₆-tagged TEV proteinase at a proteinase:sample molar ratio of 1:200. This cleavage left peptide G-A-M-D-P at the N-terminus of the protein. The digested sample was passed several times through nickel-nitrilotriacetic acid resin (Invitrogen) previously equilibrated with buffer A plus 10mM imidazole to remove His₆-containing molecules. (ii) The supernatant of the untagged protein (encoded by vector pCri7) was incubated for 1 h at 65°C and the precipitated material removed by another centrifugation step at 50,000xg for 1 h at 4°C. The supernatant was filtered (0.22 µm pore size, Millipore) and dialyzed overnight at room temperature against buffer C (20 mM Tris-HCl, 150 mM NaCl, pH 7.0). The protein was subsequently purified by cation exchange chromatography using a HiLoad 16/10 SP Sepharose HP column with a 0-1 M NaCl gradient in 10 column volumes (GE Healthcare). Protein coming from both strategy (i) and (ii) was concentrated and finally purified by size-exclusion chromatography on a HiLoad 16/60 Superdex 75 column (GE Healthcare) previously equilibrated with buffer D (20 mM Tris-HCl, 150 mM NaCl, pH 7.5). Fully active selecase was obtained after four consecutive overnight dialysis steps at 4°C against buffer C plus, respectively, 10 mM 1,10-phenantroline, 1 mM 1,10-phenantroline, 1 mM ZnCl₂, and 10 µM ZnCl₂.
4) Protein identity and purity were assessed by mass spectrometry and 15% Tricine-SDS-PAGE stained with Coomassie blue. Ultrafiltration steps were performed with Vivaspin 15 and Vivaspin 500 filter devices of 5-KDa cut-off (Sartorius Stedim Biotech). Protein concentration was determined by measuring the absorbance at 280 nm using a spectrophotometer (NanoDrop) and calculated absorption coefficientE_{0.1%}= 0.45.

### EXAMPLE 2: Proteolytic and inhibitory activity assays

Proteolytic activities were assayed at 37°C in buffer E (50 mM MES, 150 mM NaCl, pH 5.5), buffer F (50 mM HEPES, 150 mM NaCl, pH 7.5) or buffer G (50 mM CHES, 150 mM NaCl, pH 9.5) at a final protein concentration of 50 µg/ml unless otherwise stated.

Proteolytic activity against the fluorescein conjugates BODIPY FL casein, DQ gelatin, and DQ bovine serum albumin (all from Invitrogen) was measured according to the manufacturer's instructions using a microplate fluorimeter (FLx800, BioTek or Infinite M200, Tecan). Assays with natural protein substrates (at 0.5mg/ml) included bovine plasma fibronectin, bovine muscle actin, human plasma fibrinogen, cold-water fish-skin gelatin, bovine milk casein, and bovine milk α-casein (all from Sigma). Reactions were carried out in buffer F at 37°C, 65°C, and 80°C overnight and at an enzyme:substrate ratio of 1/5 (w/w) for the first two substrates and 1/10 (w/w) for the others. Cleavage was assessed by 15% Tricine-SDS-PAGE stained with Coomassie blue. Proteolytic activity was further tested on eleven fluorogenic substrates of sequence: Abz-K-D-E-S-Y-R-K(dnp) (Abz, aminobenzoyl; dnp, 2,4-dinitrophenylamino), Abz-T-V-L-E-R-S-K(dnp), Abz-D-Y-V-A-S-E-K(dnp), Abz-Y-G-K-R-V-F-K(dnp), Abz-V-K-F-Y-D-I-K(dnp), Dabcyl-L-A-R-V-E-Edans (Dabcyl, p-dimethyl(aminophenyl)azobenzoate; Edans, 2-aminoethylamino-1-naphthalene sulfonate), Abz-G-I-V-R-A-K(dnp) (Bachem), Mca-P-L-G-L-Dap(dnp)-A-R-NH₂ (Mca, 7-methoxycoumarin-4-acetyl; Dap, L-diaminopropionyl) (Bachem), Mca-R-P-K-R-V-E-Nva-W-R-K(dnp)-NH₂ (Nva, norvaline) (Bachem), Dnp-P-L-G-L-W-A-(D)R-NH₂ (Bachem), and Abz-E-L-A-Y-F-Y-P-E-K(dnp) (GL Biochem Ltd) (see [12] for details on the first six substrates). Reactions were monitored in a microplate fluorimeter (Infinite M200, Tecan) at enzyme:substrate ratios (molar/molar) of 1/0.25, 1/0.5, 1/1.25, and 1/6.25.

Esterase activity against the chromogenic substrates A-ONp (ONp, p-nitrophenyl ester), H-ONp, and L-ONp (all from Bachem), and carboxypeptidase activity against the chromogenic substrates N-(3-[2-furyl]acryloyl)-F-F-OH (Bachem), N-(3-[2-furyl]acryloyl)-G-L-A-OH (Bachem), and N-(3-[2-furyl]acryloyl)-L-G-P-A-OH (Sigma) was tested using a microplate spectrophotometer (PowerWave XS, BioTek) at enzyme:substrate ratios (molar/molar) of 1/25 and 1/125 for ester substrates and 1/5 and 1/25 for carboxypeptidase substrates. Finally, aminopeptidase activity was assayed with fluorogenic substrates F-Amc (Amc, 7-amino-4-methylcoumarin), (H)T-Amc, and Y-Amc (all from Bachem), and with the p-nitroanilide (pNA) derivatives of a representative set of natural L-amino acids and peptides (from Bachem): A-pNA, M-pNA, L-pNA, K-pNA, V-pNA, (H)I-pNA, (H)G-pNA, N-Acetyl-F-pNA, A-A-pNA, N-benzyloxycarbonyl-V-G-R-pNA, *N*(α)-benzoyl-I-E-G-R-pNA, and N-succinyl-A-A-P-F-pNA. Reactions were monitored in a microplate fluorimeter (FLx800, BioTek) or PowerWave XS spectrophotometer (BioTek) at enzyme:substrate ratios (molar/molar) of 1/125 and 1/250.

In addition, proteolytic activity was further assayed using a zymogram of bovine milk α-casein. Samples were prepared without a reducing agent or boiling. Following electrophoresis on a 12.5% Glycine-SDS-PAGE, SDS was removed from the gel with 2.5% Triton X-100. The zymogram was incubated for 48 h at 37°C in buffer F and subsequently stained with Coomassie blue. A search for the optimal pH for activity was performed with the fluorogenic substrate Abz-E-L-A-Y-F-Y-P-E-K(dnp) in the presence of 150 mM NaCl by using 50 mM MES as buffer for pH 5.5, 6.0 and 6.5; 50 mM HEPES for pH 7.0-8.0; and 50 mM CHES for pH 8.5, 9.0 and 9.5, respectively. Kinetic parameters were calculated using the same substrate in buffer F at 37°C according to the Michaelis-Menten equation implemented in the SIGMAPLOT v.10.0 program.

For inhibition assays, selecase (at 2 µM in buffer F) was incubated for 30 min with different classes of protease inhibitors and the remaining proteolytic activity was then measured using the fluorogenic substrate Abz-E-L-A-Y-F-Y-P-E-K(dnp) at 37°C. Metal substitution assays were performed by removing the pre-existing metal upon purification through dialysis against 10 mM and 1 mM 1,10-phenanthroline and subsequent dialysis against 1 mM and 10 µM chloride or sulfate salt of a set of divalent metals, similarly as described above to obtain fully active protein. Metals tested were zinc, cobalt, magnesium, manganese, calcium, cadmium, copper, and nickel.

### Results and discussion

**Proteolytic assays and inhibition tests of selecase reveal caseinolytic activity only -** Selecase proved to be soluble and was readily purified to homogeneity. The protein was properly folded as assessed by size-exclusion chromatography, which revealed that it eluted as a monomer, dimer or tetramer depending on protein concentration. Both pPROEX-HTa- and pCRI7-encoded selecase, i.e. with or without the N-terminal pentapeptide, were exclusively, selectively and specifically active against bovine αₛ₁-casein (Fig. 1A); it is therefore a selective casein peptidase (selecase). Consistent with this finding, an E⁷⁰A mutant (for amino-acid sequence, see SEQ ID No: 1) of this mature and active metallopeptidase (MP), which affects the glutamate of the zinc-binding signature that plays an essential role as a general base/acid in the catalytic process of MPs in general [13-14-15], was inactive against this substrate (Fig. 1A). Moreover, general MP inhibitors (EDTA, zinc excess, and 1,10-phenanthroline) likewise abolished activity (Fig. 1A). The MP activity was further verified by a zymogram of α-casein in the absence and in the presence of the aforementioned general MP inhibitors (Fig. 1B; similar inhibitory effects for zymograms with EDTA, zinc excess, and 1,10-phenanthroline). We determined the cleavage site of selecase on αₛ₁-casein by N-terminal Edman degradation of electro-blotted SDS-PAGE bands, which showed that cleavage occurred only between Y159 and F160 (see UP P02662). Interestingly, the cleavage sequence is reminiscent of that of TEV proteinase, which explains the partial self-cleavage of the fusion protein during purification of some preparations of selecase expressed with a TEV-cleavable N-terminal hexahistidine tag (Fig. 1 B, left gel, lane 3). Activity from contaminating TEV proteinase, which is a cysteine peptidase, was ruled out, as this protease was not active against α-casein in a control assay and it is not sensitive against MP inhibitors.

The sequence flanking the casein cleavage site led us to design a specific fluorogenic peptide (Abz-E-L-A-Y-↓-F-Y-P-E-K(dnp)) to characterize the proteolytic activity in detail (Fig. 1 C), calculate the optimum pH for activity (7.0-7.5; Fig. 1 D), and determine the corresponding kinetic parameters (k_{cat} = 4 x 10⁻⁴ s⁻¹ ± 2.1 x 10⁻⁵; Kₘ = 3 µM ± 0.3; K_{cat}/Kₘ = 130 M-¹s⁻¹ ± 14), which suggest relevant but not too potent proteolytic efficiency. Inhibitory activity assays using this fluorogenic substrate revealed complete inhibition in the presence of general MP inhibitors-as found for cleavage of α-casein (see above)-but not in the presence of general serine, cysteine or aspartic peptidase inhibitors, as expectable for an MP (Table 1). Also consistently, the E₇₀A mutant was inactive against this peptide (Fig. 1 C). Other metals than zinc were unable to restitute activity of metal-depleted apo-selecase, with the exception of cobalt, which rather enhanced its activity (Table 2). This finding is reminiscent of other MPs such as astacin [16], carboxypeptidase A [17], and thermolysin [18], whose cobalt-substituted variants were more active than the zinc forms. In conclusion, selecase is an efficient and highly specific and selective peptidase for αₛ₁-casein, which is cleaved at a single site.

| **Table 1. Inhibition of selecase activity.** | | | |
|---|---|---|---|
| Inhibitor | Concentration (mM) | Specificity | Relative activity (%) |
| None | - | - | 100 |
| ZnCl₂ | 1 | Metallopeptidases | 3 |
| EDTA | 1 | Metallopeptidases | 0 |
| PMSF | 1 | Serine peptidases | 82 |
| Pefabloc | 1 | Serine peptidases | 78 |
| Benzamidine | 1 | Serine peptidases | 66 |
| lodoacetamide | 1 | Cysteine peptidases | 79 |
| E-64 | 0.1 | Cysteine peptidases | 98 |
| Pepstatin A | 0.1 | Aspartic peptidases | 75 |

Values as mean of three independent measurements, SD within ±5

| **Table 2. Effect of divalent ions on apo-selecase reactivation.** | | |
|---|---|---|
| Cation | Concentration (mM) | Relative activity (%) |
| Zn²⁺ | 0.01 | 100 |
| Co²⁺ | 0.01 | 162 |
| Mg²⁺ | 0.01 | 4 |
| Mn²⁺ | 0.01 | 5 |
| Ca²⁺ | 0.01 | 4 |
| Cd²⁺ | 0.01 | 5 |
| Cu²⁺ | 0.01 | 16 |
| Ni²⁺ | 0.01 | 3 |

Values as mean of three independent measurements, SD within ±5

### Bibliography

[1] Rawlings ND, Barrett AJ, Bateman A. MEROPS: the database of proteolytic enzymes, their substrates and inhibitors. Nucleic Acids Res. 2012; 40: D343-50
[2] Sadat-Mekmene L, Richoux R, Aubert-Frogerais L, Madec MN, Corre C, Piot M, Jardin J, le Feunteun S, Lortal S, Gagnaire V. Lactobacillus helveticus as a tool to change proteolysis and functionality in Swiss-type cheeses. J Dairy Sci. 2013; 96: 1455-70
[3] Fox PF. Advanced Dairy Chemistry, Volume 3: Lactose, Water, Salts and Vitamins (Dairy chemistry series). Springer (2nd Edition). 1992
[4] El-Ghaish S, Rabesona H, Choiset Y, Sitohy M, Haertlé T, Chobert JM. Proteolysis by Lactobacillus fermentum IFO3956 isolated from Egyptian milk products decreases immuno-reactivity of αS1-casein. J Dairy Res. 2011; 9:1-8
[5] Audic J-L, Chaufer B, Daufin G. Non-food applications of milk components and dairy co-products: A review. Lait. 2003; 83: 417-38
[6] Vaz CM, Fossen M, van Tuil RF, de Graaf LA, Reis RL, Cunha AM. Casein and soybean protein-based thermoplastics and composites as alternative biodegradable polymers for biomedical applications. J Biomed Mater Res A. 2003; 65: 60-70.
[7] Varhimo E, Varmanen P, Fallarero A, Skogman M, Pyörälä S, livanainen A, Sukura A, Vuorela P, Savijoki K. Alpha- and β-casein components of host milk induce biofilm formation in the mastitis bacterium Streptococcus uberis. Vet Microbiol. 2011; 149: 381-9
[8] Pepe G, Tenore GC, Mastrocinque R, Stusio P, Campiglia P. Potential anticarcinogenic peptides from bovine milk. J Amino Acids. 2013; 2013: 939804
[9] Arnau J, Lauritzen C, Petersen GE, Pedersen J. Current strategies for the use of affinity tags and tag removal for the purification of recombinant proteins. Protein Expr Purif. 2006; 48: 1-13
[10] Jenny RJ, Mann KG, Lundblad RL. A critical review of the methods for cleavage of fusion proteins with thrombin and factor Xa. Protein Expr Purif. 2003; 31: 1-11
[11] Waugh DS. An overview of enzymatic reagents for the removal of affinity tags. Protein Expr Purif. 2011; 80: 283-93
[12] Botelho, T. O., Guevara, T., Marrero, A., Arêde, P., Fluxa, V. S., Reymond, J. L., Oliveira, D. C. & Gomis-Rüth, F. X. (2011). Structural and functional analyses reveal that Staphylococcus aureus antibiotic resistance factor HmrA is a zinc-dependent endopeptidase. J. Biol. Chem. 286, 25697-25709.
[13] Bayés, A., Fernández, D., Solà, M., Marrero, A., García-Piqué, S., Avilés, F. X., Vendrell, J. & Gomis-Rüth, F. X. (2007). Caught after the Act: A human A-type metallocarboxypeptidase in a product complex with a cleaved hexapeptide. Biochemistry 46, 6921-6930.
[14] Gomis-Rüth, F. X. (2008). Structure and mechanism of metallocarboxypeptidases. Crit. Rev. Biochem. Mol. Biol. 43, 319-345.
[15] Matthews, B. W. (1968). Solvent content of protein crystals. J. Mol. Biol. 33, 491-497.
[16] Gomis-Rüth, F. X., Nar, H., Grams, F., Yallouros, I., Küsthardt, U., Zwilling, R., Bode, W.& Stöcker, W. (1994). Crystal structures, spectroscopic features and catalytic properties of cobalt(II)-, copper(II)-, nickel(II)-, and mercury(II)-derivatives of the zinc-endopeptidase astacin. A correlation of structure and proteolytic activity. J. Biol. Chem. 269, 17111-17117.
[17] Auld, D. S. & Holmquist, B. (1974). Carboxypeptidase A. Differences in the mechanisms of ester and peptide hydrolysis. Biochemistry 13, 4355-4361.
[18] Holmquist, B. & Vallee, B. L. (1974). Metal substitutions and inhibition of thermolysin: spectra of the cobalt enzyme. J. Biol. Chem. 249, 4601-4607.

## Claims

1. A proteolytic enzyme **characterized in that** said protease has the amino acid sequence SEQ ID No: 1.

2. A proteolytic enzyme according to claim 1, **characterized in that** its amino acid sequence has at least 70% identity to SEQ ID No: 1.

3. A proteolytic enzyme according to claim 1 or 2, **characterized in that** said protease enzyme is obtainable by purification from cultures of *Methanococcus jannaschii.*

4. An isolated nucleic acid molecule encoding a proteolytic enzyme selected from the group consisting of:
(a) a nucleic acid molecule encoding a polypeptide comprising the amino acid sequence as depicted in SEQ ID No: 1;
(b) a nucleic acid molecule encoding a polypeptide having at least 70% identity to the amino acid sequence of SEQ ID No: 1;
(c) a nucleic acid molecule comprising the coding sequence of the nucleotide sequence as depicted in SEQ ID No: 1 plus N-terminal and C-terminal fusion proteins or tags;
(d) a nucleic acid molecule the coding sequence of which differs from the coding sequence of a nucleic acid molecule of (c) due to the degeneracy of the genetic code; and
(e) a nucleic acid molecule hybridizing under stringent conditions to a nucleic acid molecule encoding a polypeptide having an amino acid sequence which shows at least 70% identity to the amino acid sequence as depicted in SEQ ID No: 1.

5. A recombinant expression vector comprising the isolated nucleic acid molecule according to claim 4 operably linked to regulatory sequences capable of directing expression of said proteolytic enzyme encoding gene in a suitable host.

6. A host cell comprising the recombinant expression vector according to claim 5, optionally wherein said host is *Escherichia coli, Bacillus* spp., *Pichia pastoris, Saccharomyces cerevisiae,* insect and mammalian cells, or *Methanococcus* spp.

7. A process of producing a proteolytic enzyme according to any of claims 1 to 3, said process comprising the steps of culturing the host cells according to any of claims 6 or 7 and recovering the proteolytic enzyme.

8. A process for obtaining an enzyme preparation comprising the protease according to any of claims 1 to 3, said process comprising the steps of culturing a host cell according to claim 6 and either recovering said protease from the cells or separating the cells from the culture medium and obtaining the supernatant.

9. An enzyme preparation obtainable by the process according to claim 8.

10. The enzyme preparation according to claim 9, **characterized in that** said preparation comprises other enzymes selected from the group of protease, amylase, cellulase, lipase, xylanase, mannanase, cutinase, pectinase or oxidase with or without a mediator.

11. The enzyme preparation according to any of claims 9 or 10, **characterized in that** said preparation comprises a suitable additive selected from the group of stabilizers, buffers, surfactants, bleaching agents, mediators, anti-corrosion agents, builders, antiredeposition agents, optical brighteners, dyes, pigments, caustics, abrasives and preservatives.

12. Use of the proteolytic enzyme according to any of claims 1 to 3, or the enzyme preparation according to any of claims 9 to 11, in biomaterials for modifying casein-derived biomaterials properties and disruption of bacterial biofilms.

13. Use of the proteolytic enzyme according to any of claims 1 to 3, or the enzyme preparation according to any of claims 9 to 11, in the food industry for modifying the properties of milk-derived products and reduction/avoidance of allergenicity of milk/milk-derived products.

14. Use of the proteolytic enzyme according to any of claims 1 to 3, or the enzyme preparation according to any of claims 9 to 11, in the generation of novel peptides.

15. Use of the protease according to any of claims 1 to 3, or the enzyme preparation according to any of claims 9 to 11, in recombinant protein expression for removal of a fusion protein in which the peptide sequence between the fusion protein and the target protein contains this peptide sequence or related ones.
